# EUROPEAN PATENT APPLICATION

(11) **EP 2 383 013 A1**
(43) Date of publication of application: **02.11.2011**
(21) Application number: 09835035.8
(22) Date of filing: 25.12.2009
(51) Int. Cl.: A61M 37/00, A61K 9/70, A61K 38/22, A61K 47/34

(54) **MICRONEEDLE DEVICE**

(30) Priority: 26.12.2008 JP 2008334545
(71) Applicant: Hisamitsu Pharmaceutical Co., Inc., Tosu-shi, Saga 841-0017 (JP)
(72) Inventor: TOKUMOTO Seiji, Tsukuba-shi Ibaraki 305-0856 (JP); MATSUDO Toshiyuki, Tsukuba-shi Ibaraki 305-0856 (JP); KUWAHARA Tetsuji, Tsukuba-shi Ibaraki 305-0856 (JP); ISHITSUKA Shuji, Tsukuba-shi Ibaraki 305-0856 (JP)
(74) Representative: Grünecker, Kinkeldey, Stockmair & Schwanhäusser
(86) International application number: PCT/JP2009/071621
(87) International publication number: WO 2010/074239

(57) **Abstract**

The present invention aims to administer active ingredients efficiently and simply through the skin. For this purpose, a microneedle device 1 has a microneedle base 2 and microneedles 3 capable of piercing the skin and being 300 to 500 µm in height disposed on the base 2, wherein at least a part of the surface of the microneedles 3 and/or the microneedle base 2 is coated with a coating agent containing an active ingredient and a coating carrier, and a blending ratio of the active ingredient and the coating carrier is 10:0.1 to 1:10.

## Description

### Technical Field

The present invention relates to a microneedle device having a plurality of microneedles capable of piercing the skin on a base for administration of active ingredients through the skin.

### Background Art

Conventionally, a microneedle device has been known as a device for improving transdermal absorption of drugs. Microneedles disposed on a microneedle device are intended to pierce the stratum corneum, the outer most skin layer, and various sizes and shapes have been proposed. A microneedle device is desired as a non-invasive administration method (refer to Patent Literature 1).

Further, various methods have also been proposed with regard to a method of applying drugs in which a microneedle device is used. In Patent Literature 2, coating the surface of microneedles with drugs, forming a groove or a hollow part in microneedles through which drugs or body components are allowed to penetrate, mixing drugs into microneedles themselves, and the like are described. Further, in Patent Literature 2, it is also stated that it is preferable that a reservoir medium contain sugars, particularly, sugars for stabilization such as lactose, raffinose, trehalose, or sucrose, which forms glass (noncrystalline solid material).

In Patent Literatures 3 and 4, it is stated that a coating carrier of a microprojection array used for transdermal administration of a vaccine and the like is made of a biocompatible carrier composed of human albumin, polyglutamic acid, polyaspartic acid, polyhistidine, pentosan polysulfate, and polyamino acid, as well as a reducing sugar, a non-reducing sugar, and polysaccharide.

In Patent Literature 5, it is stated, as preferable characteristics of microprojections having capillary action control features, that the microprojections have a microprojection density in the range of 100 to 2000 microprojections per square centimeter and a microprojection length in the range of approximately 50 to 500 µm, and that it is preferable to apply this to the skin using an impact applicator.

### Citation List

### Patent Literature

Patent Literature 1: National Publication of International Patent Application No. 2001-506904
Patent Literature 2: National Publication of International Patent Application No. 2004-504120
Patent Literature 3: National Publication of International Patent Application No. 2004-528900
Patent Literature 4: National Publication of International Patent Application No. 2007-501070
Patent Literature 5: National Publication of International Patent Application No. 2008-534151

### Summary of Invention

### Technical Problem

Among active ingredients having physiological activities, there are many that are hard to be administered from the skin for relatively large molecular size such as proteins and peptides. The present invention was accomplished in order to solve this problem, and the present invention aims to provide a microneedle device capable of administering active ingredients efficiently and simply through the skin.

### Solution to Problem

The microneedle device of the present invention comprises a base and microneedles capable of piercing the skin and being 300 to 500 µm in height disposed on the base, wherein at least a part of the surface of the microneedles and/or the base is coated with a coating agent comprising an active ingredient and a coating carrier, and a blending ratio of the active ingredient and the coating carrier is 10:0.1 to 1:10.

According to such a microneedle device, because a coating agent containing active ingredients and coating carriers is coated, it is possible to surely coat the active ingredients on the microneedles and administer them efficiently and simply through the skin. Further, the utility of microneedles can be markedly increased.

In the microneedle device of the present invention, the active ingredient may be follicle-stimulating hormone or parathyroid hormone. In this case, it is possible to administer follicle-stimulating hormone or parathyroid hormone efficiently through the skin.

In the microneedle device of the present invention, a density of the microneedles may be 400 to 850 needles per square centimeter. In this case, it is possible to give the microneedles strength capable of piercing the skin to efficiently pierce the skin.

In the microneedle device of the present invention, a material of the microneedles may be a polylactic acid. In this case, it is possible to keep the unit price of the material low, while increasing the safety of the device.

### Advantageous Effects of Invention

According to such a microneedle device, because a coating agent containing active ingredients and coating carriers is coated, it is possible to surely coat the active ingredients on the microneedles and administer them efficiently and simply through the skin.

### Brief Description of Drawings

[Figure 1] Figure 1 is a perspective view showing one example of the microneedle device according to an embodiment.
[Figure 2] Figure 2 is a cross sectional view taken along the line II-II in Figure 1.
[Figure 3] Notations (a) to (c) are diagrams showing one example of a coating method of the microneedles.
[Figure 4] Figure 4 is a graph showing the cumulative amount penetrated in Example 1.
[Figure 5] Figure 5 is a graph showing the cumulative amount penetrated in Example 2.
[Figure 6] Figure 6 is a graph showing plasma FSH in Example 3.
[Figure 7] Figure 7 is a graph showing serum PTH in Example 4.

### Description of Embodiments

Hereinbelow, the embodiments of the present invention will be described in detail with reference to attached drawings. It is to be noted that identical signs are assigned to identical or equivalent elements and redundant description is omitted in the description of the drawings.

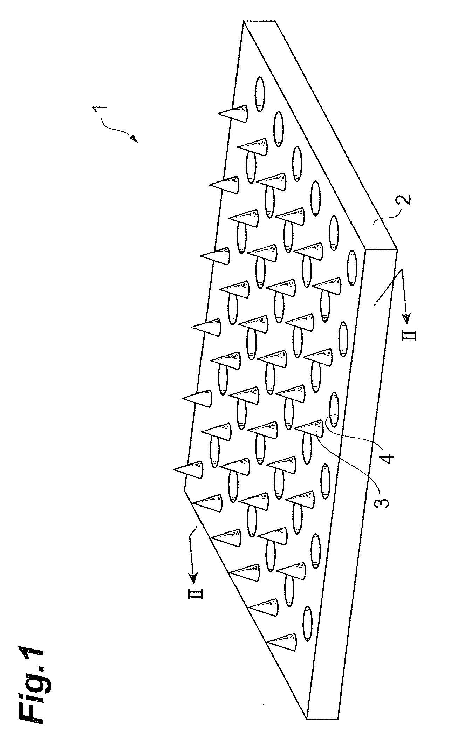

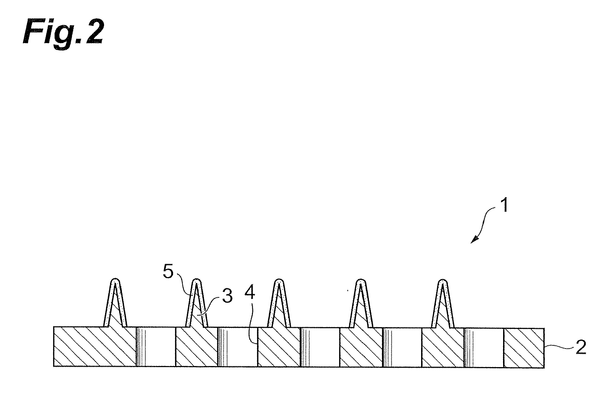

Figure 1 is a perspective view showing one example of the microneedle device according to an embodiment. Figure 2 is a cross sectional view taken along the line II-II in Figure 1.

As shown in Figure 1, a microneedle device 1 has a microneedle base 2 and a plurality of microneedles 3 capable of piercing the skin two-dimensionally arranged on the microneedle base 2.

The microneedle base 2 is a foundation to support the microneedles 3. On the microneedle base 2, a plurality of through holes 4 are formed so that they are two-dimensionally arranged. The microneedles 3 and the through holes 4 are alternately arranged in the direction of a diagonal of the microneedle base 2. By through holes 4, it becomes possible to administer physiologically active ingredients from the back of the microneedle base 2. However, a base lacking such a through hole may also be used. The area of the microneedle base 2 is 0.5 cm² to 10 cm², preferably 1 cm² to 5 cm², and more preferably 1 cm² to 3 cm². It may also be possible to configure a base of a desired size by connecting several of these microneedle bases 2.

The microneedles 3 each have a minute structure, and a height (length) thereof h is preferably 50 to 500 µm. At this point, the reason for setting the length of the microneedles 3 at 50 µm or more is to ensure transdermal administration of active ingredients, and the reason for setting the length at 500 µm or less is to avoid the contact between the microneedles and nerves so as to securely reduce the possibility of pain and securely avoid the possibility of bleeding. Also, when the length of the microneedles 3 is 500 µm or less, the amount of active ingredients to be released inside the skin can be efficiently administered. It is preferable that the length of the microneedles 3 be 300 to 500 µm, and it is particularly preferable that the length be 400 to 500 µm.

At this point, a microneedle refers to a projecting structure including, in a broad sense, a needle shape or a structure containing a needle shape. However, the microneedle is not limited to a structure having a needle shape with a tapered tip but also includes a structure lacking a tapered tip. When the microneedles 3 are in a conical shape, a diameter of the basal surface thereof is approximately 50 to 200 µm. Although the microneedles 3 are in a conical shape in the present embodiment, microneedles in a polygonal pyramid shape such as a square pyramid and the like may also be used.

As to a density of the microneedles 3, the microneedles are typically spaced apart so that a density of approximately one to 10 needles per millimeter (mm) is provided in a row of the needles. Generally, adjacent rows are spaced apart from each other by a distance substantially equal to the space between the needles in a row, and the needle density is 100 to 10000 needles per cm². When there is a needle density of 100 needles or more, the needles can efficiently pierce the skin. Meanwhile, a needle density of more than 10000 needles makes it difficult to give the microneedles 3 strength capable of piercing the skin. The density of the microneedles 3 is preferably 200 to 5000 needles, more preferably 300 to 2000 needles, and most preferably 400 to 850 needles.

While examples of a material of the microneedle base 2 or the microneedles 3 include silicon, silicon dioxide, ceramics, metals (such as stainless steel, titanium, nickel, molybdenum, chromium, and cobalt), and synthetic or natural resin materials, in consideration of the antigenicity of the microneedle and the unit price of the material, a biodegradable polymer such as polylactic acid, polyglycolide, polylactic acid-CO-polyglycolide, pullulan, capronolactone, polyurethane, and polyanhydride, and a synthetic or natural resin material such as polycarbonate, polymethacrylic acid, ethylenevinyl acetate, polytetrafluoroethylene, and polyoxymethylene, which are non-biodegradable polymers, are particularly preferable. Further, polysaccharide such as hyaluronic acid, sodium hyaluronate, dextran, dextrin, or chondroitin sulfate is also suitable.

Examples of a production method of the microneedles 3 include wet etching process or dry etching process using a silicon base, precision machining using metals or resins (such as electric discharge method, laser processing, dicing processing, hot embossing process, and injection mold processing), and machinery cutting. By these processing methods, a needle part and a support part are molded into an integrated unit. Examples of a method for hollowing the needle part include a method in which, following the production of the needle part, a secondary processing such as laser processing is performed.

On the microneedles 3, a coating 5 of a coating agent containing active ingredients and coating carriers for keeping the active ingredients is provided. The coating 5 is a coating in which a coating liquid containing active ingredients and compatible coating carriers is fixed to a part or all of the microneedles 3 and/or the microneedle base 2. The term "compatible" refers to, as far as visual assessment is concerned, that no phase separation occurs after performing centrifugation following preparation of a solution and that no formation of aggregation is observed. The term "fixed" refers to a state in which a coating liquid remains attached to an object almost uniformly. Immediately after coating, a coating liquid is fixed in a dried state by a known drying method, namely air drying, vacuum drying, freeze drying, or a combination thereof. However, after transdermal administration, the coating liquid does not necessarily remain fixed in a dried state because it may contain a water content that is in equilibrium with the surrounding atmosphere or an organic solvent.

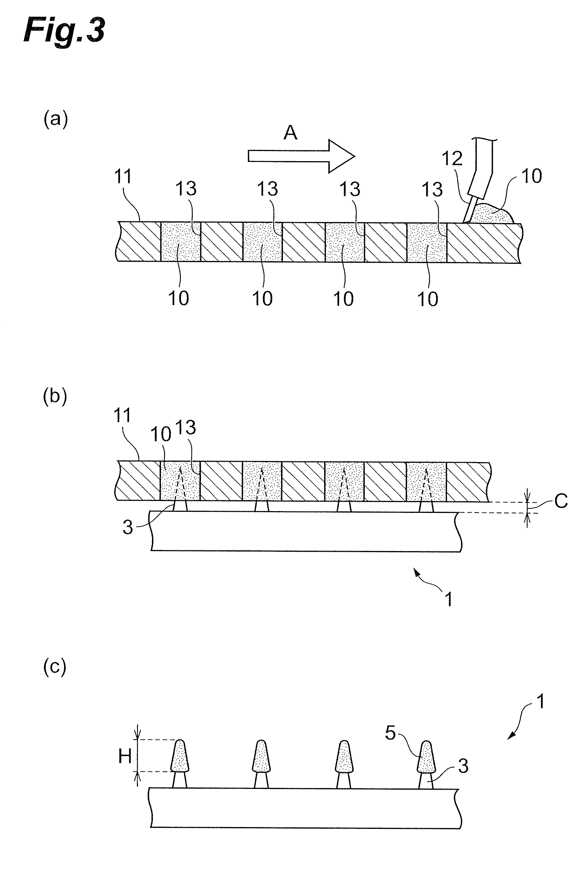

Figure 3 (a) to (c) are diagrams showing one example of a coating method of the microneedles 3. According to this method, firstly, as shown in Figure 3 (a), a coating liquid 10 is swept in the direction of an arrow A by a spatula 12 on a mask plate 11 so as to fill openings 13 with the coating liquid 10. Subsequently, as shown in Figure 3 (b), the microneedles 3 are inserted into the openings 13 of the mask plate 11. Thereafter, as shown in Figure 3 (c), the microneedles 3 are pulled out of the openings 13 of the mask plate 11. By the above operation, the coating 5 of the coating liquid 10 is provided on the microneedles 3.

A range of coating H of the microneedles 3 is controlled by clearance (gap) C shown in Figure 3 (b). This clearance C is defined as a distance between the basal surface of the microneedles 3 and the bottom surface of the mask plate 11 (base thickness is not involved), and is set according to a tension of the mask plate 11 and the length of the microneedles 3. A range of the distance of clearance C is preferably 0 to 500 µm. When the distance of clearance C is 0, the whole of the microneedles 3 is coated. Although the range of coating H varies depending on the height of the microneedles 3 h, it may be set at 0 to 500 µm, and it is normally 10 to 500 µm, and preferably approximately 30 to 300 µm.

A thickness of the coating 5 of the microneedles 3 is less than 50 µm, preferably less than 25 µm, and more preferably 1 to 10 µm. Generally, the thickness of coating is an average thickness as measured over the surface of the microneedles 3 after drying. The thickness of coating can generally be increased by applying multiple films of the coating carrier, namely, by repeating a coating process after fixation of the coating carrier.

When coating is performed on the microneedles 3, in order to minimize changes in drug concentrations and physical properties caused by volatilization of a solvent of the coating agent, it is preferable to control temperature and humidity in an installation environment of an apparatus at a constant level. In order to prevent solvent evaporation, it is preferable to either decrease the temperature or increase the humidity, or control both. The humidity at room temperature when the temperature is not controlled is, as a relative humidity, 50 to 100% RH, preferably 70.0 to 99.9% RH. When the humidity is 50% RH or less, solvent evaporation occurs, causing physical properties of a coating solution to change. Although a humidification method includes a gas system, a steam vapor system, a water spray system, and the like, no particular limitation is imposed thereon as long as an intended humidity condition is assured. As a thickening agent mixed into the coating solution, it is preferable to select pullulan, which has high wettability and moisture retaining properties that minimize the volatility of the solvent.

The coating agent contains physiologically active ingredients and purified water and/or coating carriers. There are low molecular weight coating carriers and high molecular weight coating carriers as the coating carrier. Examples of the low molecular weight coating carrier include proline, trehalose, sucrose, lactose, fructose, galactose, mannose, maltose, glucose, mannitol, isopropanol, propanol, butanol, propylene glycol, and glycerin. Examples of the high molecular weight coating carrier include polyethylene oxide, polyhydroxymethylcellulose, polyhydroxypropylcellulose, polyhydroxypropylmethylcellulose, polymethylcellulose, dextran, polyethylene glycol, polyvinyl alcohol, polyvinyl pyrrolidone, pullulan, carmellose sodium, chondroitin sulfate, hyaluronic acid, sodium hyaluronate, dextrin, and gum arabic.

Also, a coating carrier of sugars that is relatively compatible (having the property of being homogeneously mixed) with proteins or peptides among other active ingredients is preferable. Specifically, trehalose, sucrose, lactose, fructose, galactose, mannose, maltose, glucose, mannitol, polyhydroxymethylcellulose, polyhydroxypropylcellulose, polyhydroxypropylmethylcellulose, polymethylcellulose, dextran, polyethylene glycol, pullulan, carmellose sodium, chondroitin sulfate, hyaluronic acid, sodium hyaluronate, dextran, and gum arabic, and the like are preferable, and further, polyhydroxypropylcellulose, pullulan, and gum arabic are more preferable. Particularly, pullulan and sucrose are preferable. In another example, examples of the coating carrier that is compatible (having the property of being homogeneously mixed) with proteins or peptides among other active ingredients include polyvinyl pyrrolidone, polyvinyl alcohol, carboxyvinyl polymer, polyacrylic acid, sodium polyacrylate, polyethylene oxide, and polyethylene glycol.

A content of the coating carrier in the coating agent is 1 to 90% by weight, preferably 1 to 70% by weight, and particularly preferably 10 to 65% by weight. Also, the coating carrier may have to have a certain degree of viscosity so as not to drip, and a viscosity of approximately 100 to 100000 cps is necessary. A more preferable viscosity is 500 to 50000 cps. For the viscosity being within the above range, it becomes possible to apply a desired amount of a coating solution at once without depending on the material of the microneedles 3. Also, generally, there is a tendency that the higher the viscosity, the larger the amount of a coating solution.

A liquid composition used for coating the microneedles 3 is prepared by mixing biocompatible carriers, beneficial active ingredients to be delivered, and in some cases, any of coating aids with a volatile liquid. The volatile liquid can be water, dimethyl sulfoxide, dimethyl formamide, ethanol, isopropyl alcohol, and a mixture thereof. Among them, water is most preferable. A coating solution in a liquid state or a suspension can typically have a concentration of beneficial physiologically active ingredients of 0.1 to 65% by weight, preferably 1 to 40% by weight, more preferably 10 to 30% by weight. The coating is particularly preferably in a fixed state. A surfactant can be zwitter ion type, amphoteric ion type, cationic type, anionic type, or nonionic type. For example, the surfactant can be Tween 20 and Tween 80, other sorbitan derivatives such as sorbitan laurate, and alkoxylated alcohols such as Laureth-4. For example, in order to dissolve a larger amount of high molecular weight active ingredients into the coating carrier or to coat a larger amount of high molecular weight active ingredients on the needles, adding surfactants is also effective.

Other known pharmaceutical aids may be added to the coating as long as they do not adversely affect a necessary solubility and characteristics of the viscosity of the coating as well as physical integrity of the dried coating.

Although no particular limitation is imposed on the active ingredient used in the present invention, high molecular weight compounds such as proteins and peptides having relatively large molecular weights or derivatives thereof are suitable. High molecular weight refers to, as an index, a molecular weight of 1000 or more, and an upper limit of the molecular weight is not particularly set. Examples include α-interferon, β-interferon for multiple sclerosis, erythropoietin, follicle stimulating hormone (FSH), follitropin β, follitropin α, G-CSF, GM-CSF, human chorionic gonadotropin, leutinizing hormone, salmon calcitonin, glucagon, GNRH antagonist, insulin, human growth hormone, filgrastim, parathyroid hormone (PTH), and somatropin. Also, examples of a vaccine include influenza vaccine, Japanese encephalitis vaccine, rotavirus vaccine, Alzheimer's disease vaccine, arteriosclerosis vaccine, cancer vaccine, nicotine vaccine, diphtheria vaccine, tetanus vaccine, pertussis vaccine, Lyme disease vaccine, rabies vaccine, diplococcus pneumoniae vaccine, yellow fever vaccine, cholera vaccine, vaccinia vaccine, tuberculosis vaccine, rubella vaccine, measles vaccine, mumps vaccine, botulism vaccine, herpes vaccine, other DNA vaccines, and hepatitis B vaccine. Particularly, follicle stimulating hormone (FSH) and parathyroid hormone (PTH) are preferable.

It is to be noted that these drugs may be used singly or two or more kinds thereof can be used in combination, and they exist in a free form or as pharmaceutically acceptable salts. As long as the salt is pharmaceutically acceptable, needless to say, a drug in a form of either an inorganic salt or an organic salt is encompassed. Also, while basically the drug is contained in the coating carrier, it is also possible to prepare a coating carrier lacking the drug and later supply the drug through the through holes 4 formed in the microneedle base 2.

The active ingredient and the coating carrier are generally blended in a ratio of 1:10 to 10:0.1, preferably in a ratio of 1:8 to 8:1, and more preferably in a ratio of 1:3 to 8:1. As one example, the blending ratio of pullulan, suitable as a coating carrier, and follicle stimulating hormone, an active ingredient, is 1:2.

As an administration method using the microneedle device 1, direct administration by hand-pushing or a method in which administration is conducted by hand-pushing with the use of a simple supplemental device for fixing the microneedle device 1 may be possible. In either way of administration, when the microneedle device 1 is contacted with the skin, it is administered by a force of 1.0 to 10 kg, preferably by a force of 1.0 to 7 kg, and more preferably by a force of 1.0 to 4 kg. Also, administration time at such a force is not so long, and it is from several seconds to several minutes at longest, and depending on the case, instant administration that takes less than a second is also possible. However, it is also possible to fix the microneedle device 1 on the skin thereafter for continuous administration of active ingredients. Further, it is also possible to administer the microneedle device 1 using a device generating collision energy.

### Examples

Hereinbelow, the present invention will be specifically described based on Examples; however, the present invention is not limited in any way to these Examples.

### (Example 1) In vitro test by microneedles (follicle-stimulating hormone)

### <Human skin penetration test>

Three types of microneedles made of a polylactic acid that differ in a length of a needle part (h: 300 µm, 400 µm, and 500 µm) were used. A shape of any of these three types of microneedles is square pyramid, and a density of the microneedles was 400 needles/cm². Also, an area of any of microneedle devices was 1 cm².

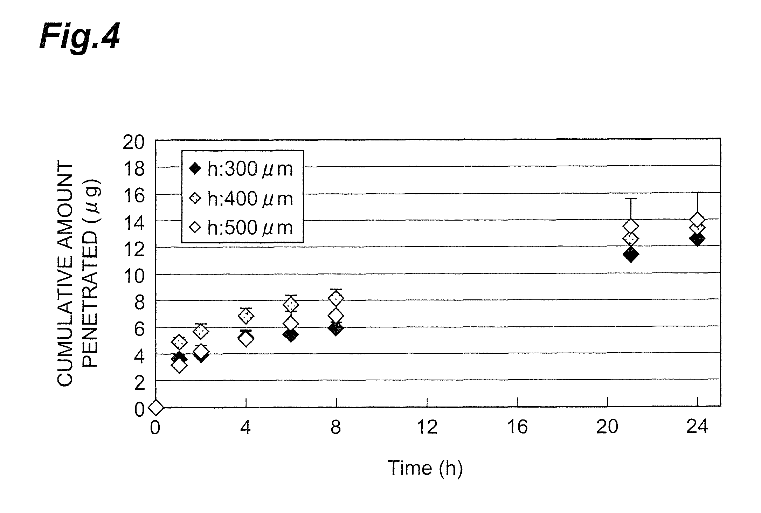

A liquid obtained by adding 26.4 µL of [¹²⁵I]FSHaq concentrated 10-fold with Biomax (molecular weight fraction: 10,000), 14.4 mg of Cold FSH (the product of ProSpec-TechnoGene Ltd), and 7.2 mg of pullulan into an Eppendorf tube and dissolving the content by centrifugation was provided as a coating liquid (30% follicle-stimulating hormone, 15% pullulan). The coating liquid thus obtained (30% follicle-stimulating hormone, 15% pullulan) was coated on the microneedle device by the aforementioned coating method (metal mask standard: one side of an aperture 220 µm, thickness 100 µm, and humidity at room temperature 85% RH or more). Subsequently, after piercing excised human skin with the microneedle device thus coated by finger pressing (2 kg/patch), the device and the skin were placed on an acrylic cell for sampling over time. As a receptor layer, phosphate buffered saline (PBS) was used, and after mixing and stirring 1 mL of a receptor liquid obtained per sampling time and acetonitrile at 1:1 and centrifuging this mixture (15,000 rpm, 5°C, 5 min), a supernatant was collected and an FSH concentration was measured by a γ-counter as described below. The results are as shown in the graph in Figure 4.

An amount of coating was calculated by using a microneedle device to which 1 to 5 µL of 10 times concentrated [¹²⁵I]FSHaq was added dropwise as a calibration curve by a γ-counter and a NaI counter. The amount of FSH coated on the microneedles was approximately 30 µg.

### (Example 2) In vitro human skin penetration test by microneedles (parathyroid hormone)

### <Human skin penetration test>

Two types of microneedles made of a polylactic acid that differ in the length of the needle part (h: 300 um and 500 µm) were used. The shape of either of these two types of microneedles was square pyramid, and the density of the microneedle was 400 needles/cm². Also, the area of either one of microneedle devices was 1 cm².

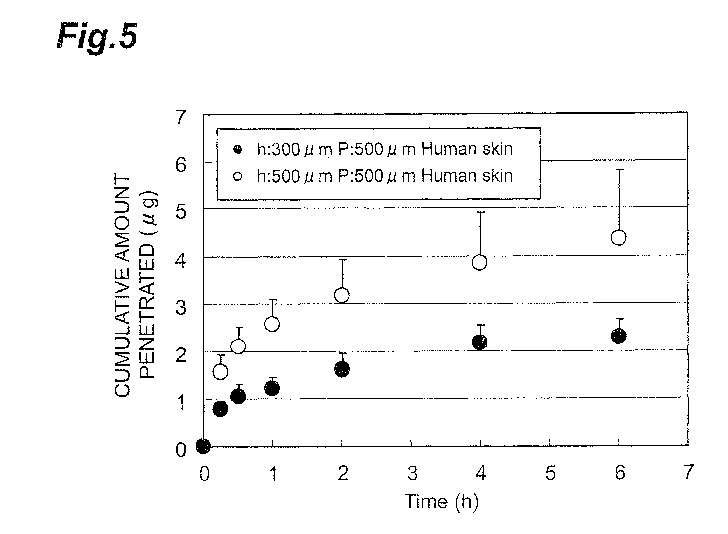

A coating liquid prepared according to the below-described technique (30% human parathyroid hormone hPTH (1-34) (the product of Sichuan Unibiochem Co., Ltd.), 7.5% pullulan, and 0.1 % Tween 20) was coated on a microneedle device similarly to Example 1, and after piercing excised human skin with this microneedle device by finger pressing (2 kg/patch), the device and the skin were placed on an acrylic cell for sampling over time. As a receptor layer, physiological saline containing 0.01% benzalkonium chloride was used, and 1 mL of a receptor liquid collected per sampling time was measured by a gamma counter to calculate a PTH concentration. It is to be noted that a period of attachment of the microneedle device was set at six hours. The results of the sampling are as shown in the graph in Figure 5.

Methods for preparing a coating solution and measuring the amount of coating
(1) Into a solution containing ¹²⁵I-PTH, hPTH (1-34), pullulan, and Tween were added so that each accounted for 30%, 7.5%, and 0.1%, respectively, and after stirring, the content was dissolved by a centrifugation method.
(2) After coating microneedles with the coating solution prepared in (1), radiation of each pharmaceutical preparation was counted using a NaI counter to estimate the content of PTH coated on the needle part. It is to be noted that the amount of coating on the microneedles was set at 40 µg in terms of an hPTH content.

As a result, comparing a height of 300 µm and a height of 500 µm, no difference was observed in the hPTH content; however, there was a difference in the skin penetration property, and it was revealed that efficient drug delivery is made possible by the use of microneedles having a height of 500 µm.

### (Example 3) In vivo absorption test by microneedles (follicle-stimulating hormone)

### <Hairless rat in vivo administration test>

Using a total of four types of microneedles, namely microneedles made of a polylactic acid having a density of 841 needles/cm² and a length of the needle part of 300 µm and three types of microneedles made of a polylactic acid that has a density of 400 needles/cm² in common but differ in the length of the needle part (h: 300 µm, 400 µm, and 500 µm), a hairless rat in vivo absorption test was conducted. The shape of any of these four types of microneedles is square pyramid, and the area of each microneedle device was 1 cm².

Firstly, the abdomen of a hairless rat was pierced with a microneedle device in which tips of the needles were coated with a similar coating liquid to Example 1 (30% FSH, 15% pullulan) by a similar technique to Example 1 by hand pushing. Then, plasma was collected over time (amount of blood collected: 300 µL/time) to measure the FSH concentration with a SPAC-S FSH kit (the product of TFB, Inc.) Also, in a subcutaneous administration group, 300 µL of 1 IU/mL FSH (physiological saline solution) was subcutaneously administered to the back of a hairless rat and the FSH concentration was similarly measured. It is to be noted that the time of attachment of the microneedle device was set at two hours.

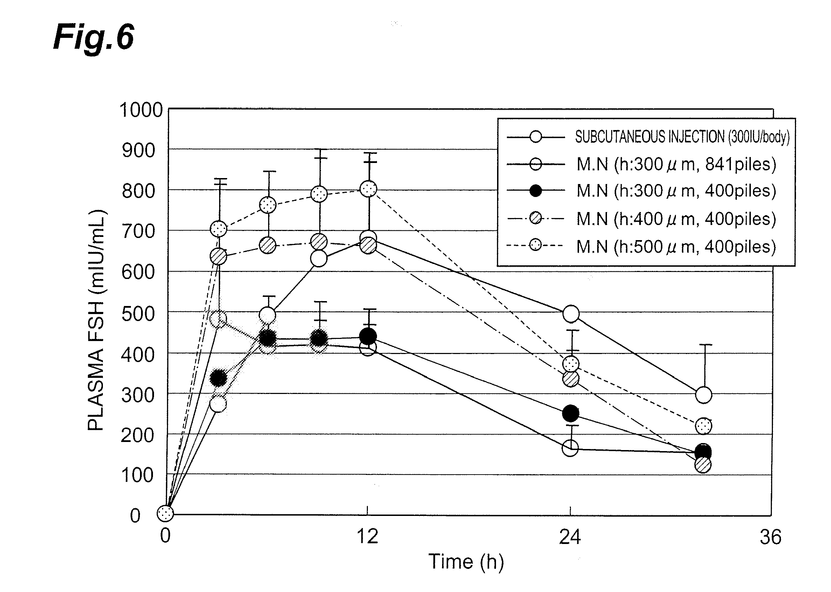

By this test, it was revealed that efficient administration of FSH became possible as the length of the microneedle increased, in the order of 300 µm < 400 µm < 500 µm (refer to Figure 6). Also, when compared at a length of 300 µm, it was found that the density of the microneedle did not cause changes in the amount of FSH administered very much. Also, when a microneedle device is used, an initial rise in the blood FSH concentration was faster than subcutaneous administration, rapidly reaching the maximum blood concentration.

### (Example 4) In vivo absorption test by microneedles (parathyroid hormone)

### <Hairless rat in vivo administration test>

Two types of microneedles made of a polylactic acid that differ in the length of the needle part (h: 300 µm and 500 µm) were used. The shape of either of these two types of microneedles was square pyramid, and the density of the microneedle was 400 needles/cm². Also, the area of either one of microneedle devices was 1 cm².

Firstly, the abdomen of a hairless rat was pierced with a microneedle device in which tips of the needles were coated with a similar coating liquid to Example 2 (30% human parathyroid hormone hPTH (1-34) (the product of Sichuan Unibiochem Co., Ltd.), 7.5% pullulan, and 0.1% Tween 20) by a similar technique to Example 1 by hand pushing, and the microneedle device was covered with a foam tape (the product of 3M Company) and left attached for six hours. Then, blood was collected over time (amount of blood collected 200 µL/times) to measure the serum hPTH concentration with a Rat PTH IRMA kit (the product of Immutopics International). Also, in a subcutaneous administration group, 0.2 mg/mL hPTH in physiological saline was prepared, 40 µL of which was subcutaneously administered to the back of a hairless rat, and the PTH concentration was similarly measured.

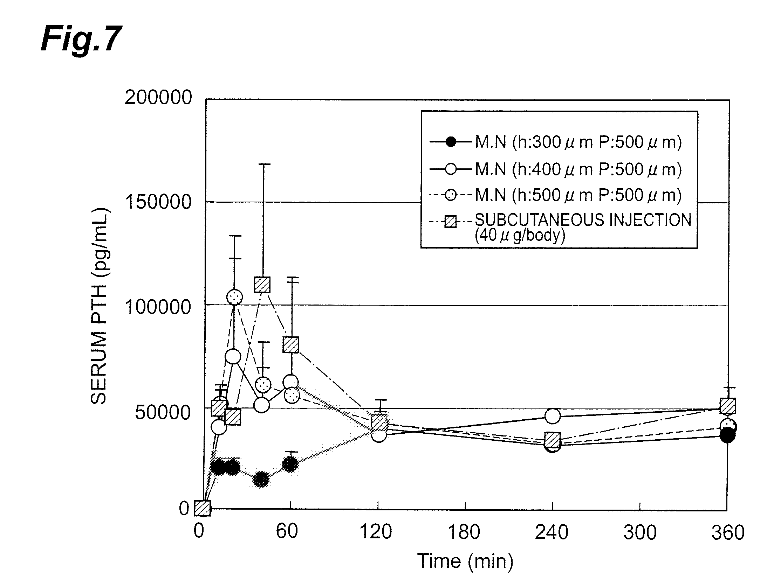

Also from this test, increases in the amount of absorption dependent on the length of the microneedles were confirmed, revealing that more efficient PTH administration becomes possible with longer needles (refer to Figure 7). Particularly, efficient administration is made possible by the use of microneedles having a length of the needle of 400 µm or more, and about the initial rise in the blood concentration, the maximum blood concentration was more rapidly reached than subcutaneous administration.

### (Example 5) A primary skin irritation study in rabbits

Three types of microneedles made of a polylactic acid that differ in the length of the needle part (h: 300 µm, 400 µm, and 500 µm) were prepared. The shape of any of these three types of microneedles was square pyramid, and the density of any of them was 400 needles/cm², and the area of any of them was 1 cm². Using these microneedles, assessment of skin response was conducted in accordance with the method of Draize. That is, after pressing a test substance to a part of the shaven back of an 18-week-old female white rabbit (KB1:JW) by a force of 3 kg/patch for 5 seconds, the test substance was attached for two hours (with a material for covering). Then, two hours after administration, the test substance was peeled off, and 0.5, 2, 24, 48, and 72 hours after peeling off, formation of erythema/crust and edema was visually observed, followed by scoring based on the assessment criteria of Draize., et al. (Table 1).

Judgment of skin primary irritation was conducted by calculating a Primary Irritation Index; P.I.I. and using the below-described assessment criteria of Draize (Table 2). The Primary Irritation Index was calculating by obtaining an average assessment score on formation of erythema and edema 0.5 hour, 24 hours, and 48 hours after peeling of the test substance in each individual, and further obtaining the sum of the average assessment score in each group, and then dividing the resulting number by the number of individuals.

The Primary Irritation Indexes (P.I.I) of the three kinds of microneedles that differ in the height of the needle part (300 µm, 400 µm, and 500 µm) were 0.3, 0.5, and 0.5, respectively, and while they were determined as mildly irritating substances, they showed a tendency of increasing skin irritating property corresponding to the height of the needle.

**[Table 1]**

| Skin response assessment criteria (Draize) | |
|---|---|
| Formation of erythema and crust | Assessment score |
| No erythema | 0 |
| Very mild erythema (barely recognizable) | 1 |
| Clear erythema | 2 |
| Moderate to severe erythema | 3 |
| Severe erythema (beet red) to formation of slight crust (deep-seated damage) | 4 |

| Formation of edema | Assessment score |
|---|---|
| No edema | 0 |
| Very mild edema (barely recognizable) | 1 |
| Mild edema (Clear edge is recognizable by distinctive protrusion) | 2 |
| Moderate edema (protrusion of about 1 mm) | 3 |
| Severe edema (protrusion of about 1 mm with expansion beyond the exposed area) | 4 |

**[Table 2]**

| Assessment criteria for skin primary irritation (Draize) | |
|---|---|
| P.I.I. ≤ 2 | Mildly irritating substance |
| 2 < P.I.I. ≤ 5 | Moderately irritating substance |
| 5 < P.I.I. ≤ 8 | Strongly irritating substance |

### (Example 6) parathyroid hormone (PTH) storage method

PTH was prepared at 40 mg/mL with water for injection, which was then dispensed to serve as a drug solution. It was further diluted to 20 mg/mL with water for injection before experiment, 20 µL of which was added dropwise to a microneedle base (400 µg/patch), which served as a medicinal unit.

For screening of a stabilizing agent, a stabilizing agent is dissolved in water for injection to prepare a 50% solution (mannitol, glycine, and citric acid were 20%), which was mixed with a 40 mg/mL PTH solution at 1:1 to serve as a drug solution.

A medicinal unit sample for screening of a stabilizing agent was fully dried and enclosed in an aluminum packing material, sandwiched between silicon treated liners, with OZO (OZO kagakugiken Co., Ltd.). It was then stored at 25°C, 40°C, 50°C, and 60°C to perform an acceleration test to conduct a stability test of the content over time (n = 3). The content was returned to room temperature, and then extracted with 4 mL of an extraction liquid, followed by measurement by HPLC.

### <Conditions>

Extraction liquid: a 0.01% benzalkonium chloride solution or 2% TMTDAB, 1% sodium chloride, and 10 mM acetic acid buffer
Detector: an ultraviolet absorptiometer (measurement wavelength: 220 nm)
Column: YMC-PackODS-AM (YMC Co., Ltd.)
Column temperature: 25°C

**[Table 3]**

| | | | 1W | 2W | 4W |
|---|---|---|---|---|---|
| Form of package | Only PTH | 40°C | 100.22 ± 0.53 | 97.64 ± 0.72 | 97.02 ± 0.65 |
| | | 50°C | 94.65 ± 1.55 | 94.10 ± 2.25 | 84.26 ± 1.19 |
| | | 60°C | 89.50 ± 1.11 | 88.22 ± 0.82 | 71.80 ± 1.94 |
| | OZO | 40°C | 104.85 ± 0.61 | 100.70 ± 1.32 | 98.52 ± 0.84 |
| | | 50°C | 97.40 ± 0.77 | 97.88 ± 1.49 | 90.64 ± 0.42 |
| | | 60°C | 92.62 ± 1.20 | 84.93 ± 4.69 | 79.21 ± 1.28 |
| | AGELESS | 40°C | 100.65 ± 3.98 | 99.75 ± 1.04 | 97.37 ± 0.76 |
| | | 50°C | 95.60 ± 0.89 | 95.73 ± 2.31 | 83.82 ± 0.37 |
| | | 60°C | 82.76 ± 1.24 | 81.58 ± 4.55 | 54.25 ± 1.24 |
| | Vacuum | 40°C | 102.65 ± 1.48 | 98.73 ± 1.70 | 95.73 ± 1.31 |
| | | 50°C | 93.76 ± 0.74 | 90.17 ± 4.85 | 82.95 ± 0.53 |
| | | 60°C | 88.71 ± 1.68 | 89.14 ± 1.87 | 62.33 ± 12.3 |
| Stabilizing agent | Trehalose | 40°C | 101.72 ± 1.06 | 101.62 ± 1.11 | 97.63 ± 1.06 |
| | (5 mg) | 50°C | 97.66 ± 1.72 | 97.92 ± 1.30 | 89.96 ± 0.60 |
| | | 60°C | 92.79 ± 1.45 | 90.56 ± 1.47 | 79.22 ± 4.24 |
| | Sorbitol | 40°C | 103.53 ± 1.90 | 97.09 ± 1.51 | 95.22 ± 0.74 |
| | (5 mg) | 50°C | 90.17 ± 1.79 | 89.04 ± 0.3 | 27.51 ± 0.91 |
| | | 60°C | 75.40 ± 2.58 | 69.13 ± 0.73 | 51.10 ± 0.84 |
| | Mannitol | 40°C | 105.42 ± 1.42 | 102.66 ± 0.94 | 98.03 ± 2.17 |
| | (2mug) | 50°C | 99.24 ± 0.48 | 99.40 ± 0.37 | 89.43 ± 1.30 |
| | | 60°C | 95.42 ± 1.80 | 93.76 ± 1.04 | 79.67 ± 2.20 |
| | Sucrose | 40°C | 102.86 ± 0.51 | 97.93 ± 2.89 | 99.20 ± 0.56 |
| | (5 mg) | 50°C | 99.83 ± 1.18 | 101.38 ± 1.42 | 92.61 ± 0.52 |
| | | 60°C | 96.71 ± 1.68 | 96.85 ± 0.32 | 82.14 ± 3.97 |
| | Proline | 40°C | 101.97 ± 2.02 | 101.40 ± 2.00 | 96.70 ± 0.82 |
| | (5 mg) | 50°C | 98.18 ± 0.38 | 101.06 ± 1.54 | 92.23 ± 0.36 |
| | | 60°C | 95.10 ± 1.00 | 93.97 ± 0.32 | 80.98 ± 1.25 |
| | Glycine | 40°C | 104.17 ± 0.37 | 100.12 ± 0.15 | 98.41 ± 0.95 |
| | (2 mg) | 50°C | 96.00 ± 2.75 | 99.51 ± 1.24 | 90.14 ± 0.53 |
| | | 60°C | 91.56 ± 1.50 | 91.87 ± 1.55 | 80.59 ± 1.24 |
| | Citric acid | 40°C | 42.53 ± 0.86 | - | - |
| | (2 mg) | 50°C | 33.95 ± 0.74 | - | - |
| | | 60°C | 0.07 ± 0.02 | - | - |

| | | | | | |
|---|---|---|---|---|---|
| Each value is expressed as the residual ratio (%) relative to the initial value (n = 3) | | | | | |

OZO (a moisture adsorbent/desiccant supplied by OZO kagakugiken Co., Ltd.) was more effective than AGELESS (an oxygen absorber manufactured by Mitsubishi Gas Chemical Company, Inc.) for storage of PTH, and sucrose was most effective as a stabilizing agent. By these results, using sucrose as the coating agent of PTH to the microneedles and storing the microneedle device in an aluminum packing material with OZO can be considered.

### (Example 7) Study on the addition amount of sucrose to parathyroid hormone (PTH)

Sucrose was dissolved in water for injection to prepare each of a 10% sucrose (1 mg) solution, a 5% sucrose (0.5 mg) solution, and a 1% sucrose (0.1 mg) solution, which was mixed with a 40 mg/mL PTH solution at 1:1 to serve as a drug solution. Other than this, the study was conducted in the same manner as the aforementioned Examples.

**[Table 4]**

| | | 1W | 4W |
|---|---|---|---|
| PTH | 40°C | | 92.82 ± 0.74 |
| | 50°C | | 80.83 ± 4.57 |
| | 60°C | 83.26 ± 0.57 | 76.55 ± 3.95 |
| Sucrose | 40°C | | 95.09 ± 0.27 |
| (1 mg) | 50°C | | 87.65 ± 4.68 |
| | 60°C | 86.99 ± 2.42 | 85.39 ± 1.07 |
| Sucrose | 40°C | | 95.34 ± 1.22 |
| (0.5 mg) | 50°C | | 89.43 ± 1.67 |
| | 60°C | 86.10 ± 1.22 | 82.27 ± 1.19 |
| Sucrose | 40°C | | 93.58 ± 1.24 |
| (0.1 mg) | 50°C | | 86.96 ± 0.59 |
| | 60°C | 85.79 ± 1.46 | 77.83 ± 1.01 |

| | | | |
|---|---|---|---|
| Each value is expressed as the residual ratio (%) relative to the initial value (n = 3) | | | |

As shown in the above Table 4, based on an observation that a microneedle device has exhibited its effect even when the addition amount of sucrose is 0.1 mg, it is understood that the microneedle device exerts its effect as long as a ratio of the drug and sucrose is 1:0.25 or more.

### (Example 8) Study on drying time of a sucrose additive to parathyroid hormone (PTH)

Sucrose was dissolved in water for injection to prepare a 10% sucrose (1 mg) solution, which was mixed with a 40 mg/mL PTH solution at 1:1 to serve as a drug solution. Using this solution, a drug solution unit was produced, and preservation qualities resulting from various drying times were examined under a nitrogen stream.

**[Table 5]**

| Drying time | 60°C | 1W | 4W |
|---|---|---|---|
| 15 minutes | Only PTH | 85.35 ± 0.64 | 75.43 ± 1.69 |
| | Sucrose | 90.31 ± 1.13 | 84.56 ± 1.60 |
| 30 minutes | Only PTH | 85.53 ± 0.49 | 73.10 ± 0.36 |
| | Sucrose | 92.71 ± 0.66 | 86.34 ± 1.28 |
| | | | |
| 1 hour | Only PTH | 84.62 ± 0.72 | 75.56 ± 1.69 |
| | Sucrose | 92.26 ± 1.04 | 85.94 ± 0.65 |
| | | | |
| 2 hours | Only PTH | 86.01 ± 1.07 | 73.93 ± 1.41 |
| | Sucrose | 95.22 ± 2.42 | 90.51 ± 4.47 |
| | | | |
| 16 hours | Only PTH | 87.08 ± 0.72 | 72.71 ± 2.01 |
| | Sucrose | 96.38 ± 2.44 | 89.37 ± 1.59 |
| | | | |

| | | | |
|---|---|---|---|
| Each value is expressed as the residual ratio (%) relative to the initial value (n = 3) | | | |

As shown in the above Table 5, it was revealed that approximately two hours are enough for the effect of drying time of the drug solution unit, and it was found that approximately two hours of drying was necessary also when the drug solution unit was applied to the microneedles.

### (Example 9)

In order to optimize a ratio between PTH (1-34) (the product of Sichuan Unibiochem Co., Ltd.) and a coating carrier, which is a water-soluble high molecular weight polymer, a study was conducted using PTH and a coating carrier (pullulan). As shown in Table 6, solutions were prepared so that concentrations of PTH were 0 to 40% (w/w). The concentration of pullulan was adjusted based on the viscosity of the solution at the time of dissolving, and the range of the concentration of pullulan was set to 0 to 30% (w/w). It is to be noted that, as the pullulan, one recited in Japanese Pharmacopeia (the product of Hayashibara Biochemical Labs., Inc.) was used. Further, in order to increase the adhesiveness to the needles, a study was also conducted for the case in which a surfactant (Tween 20, 0.1 %) was added as shown in Table 7.

Evaluation of solubility of PTH was performed after adjusting each solution, and a coating liquid capable of adjusting a uniform solution was subjected to an operation for coating of microneedles made of a polylactic acid. Following the aforementioned method as a coating method, coating was performed only on the tip part of the needles with the use of a metal mask (only the needle part was coated using a metal mask having a diameter of the aperture: 220 µm and thickness: 100 µm).

**[Table 6]**

| | | PTH (%) | | | |
|---|---|---|---|---|---|
| | | 10 | 20 | 30 | 40 |
| | 0 | 0 | 5.2 ± 2.9 | 10.7 ± 1.8 | (Gelled ×) |
| | 5 | 4.2 ± 1.9 | 8.1 ± 0.4 | 19.4 ± 2.9 | (Gelled ×) |
| Pullulan (%) | 10 | 6.7 ± 0.3 | 10.1 ± 1.4 | 8.7 ± 2.3 | (Solubility ×) |
| | 20 | 8.2 ± 0.9 | 11.7 ± 1.6 | (Solubility ×) | (Solubility ×) |
| | 30 | 10.4 ± 0.7 | (Gelled ×) | (Solubility ×) | (Solubility ×) |

**[Table 7]**

| | | PTH (%) + Tween 20 (%) | |
|---|---|---|---|
| | | 30 (0.1 Tw) | 40 (0.1 Tw) |
| | 0 | - | - |
| | 5 | | 26.3 ± 6.1 |
| Pullulan (%) | 7.5 | 23.4 ± 4.3 | (Gelled ×) |
| | 10 | 24.2 ± 9.7 | (Gelled ×) |

As a result, when pullulan was used as the coating carrier, there was a tendency that, as long as the concentration of PTH is 20% or less, the amount of PTH coated increased corresponding to the addition amount of pullulan. Also, when the concentration of pullulan was 20% or less, there was a tendency that the amount of PTH coated also increased as the concentration of PTH increased. In contrast, when the concentration of PTH was set at 30%, there was no tendency that the amount of PTH coated increased depending on the concentration of pullulan. As a reason for this, an increased surface tension of coating liquid attributable to addition of high concentration of PTH (decreased adhesiveness) is considered. Indeed, regarding a 30% or 40% PTH solution, an improvement was noted in the amount of coating when a small amount of surfactant (Tween 20) was added, and it became possible to attach 20 µg or more PTH by single coating operation with either a 30% or 40% PTH solution.

Taking a clinical dose of PTH (10 µg or more) into consideration, a ratio of PTH and pullulan is preferably 1:3 to 8:1 and the concentration of PTH at the time of adjusting a coating solution is preferably 10% or more.

### Industrial Applicability

According to the present invention, it is possible to uniformly coat active ingredients on microneedles. Also, since a solution is uniform, a highly precise coating control can be exerted on the microneedles. Furthermore, since it is possible to efficiently administer active ingredients from the skin by a microneedle device and markedly increase the utility of a microneedle device, the present invention has industrial applicability.

### Reference signs List

1···microneedle device, 2···microneedle base, 3···microneedle, 4···through hole, and 5···coating

## Claims

1. A microneedle device comprising:
a base,
microneedles capable of piercing a skin and being 300 to 500 µm in height disposed on the base,
wherein at least a part of a surface of the microneedles and/or the base is coated with a coating agent comprising an active ingredient and a coating carrier,
and a blending ratio of the active ingredient and the coating carrier is 10:0.1 to 1:10.

2. The microneedle device according to claim 1, wherein the active ingredient is follicle-stimulating hormone or parathyroid hormone.

3. The microneedle device according to claim 1 or 2, wherein a density of the microneedles is 400 to 850 needles per square centimeter.

4. The microneedle device according to any one of claims 1 to 3, wherein a material of the microneedles is a polylactic acid.
